# EUROPÄISCHE PATENTANMELDUNG

(11) **EP 1 818 056 A1**
(43) Veröffentlichungstag der Anmeldung: **15.08.2007**
(21) Anmeldenummer: 07105299.7
(22) Anmeldetag: 14.01.2004
(51) Int. Cl.: A61K 31/41, A61K 31/366, A61P 9/10

(54) **Pharmazeutische Kombination zur Prophylaxe oder Therapie von kardiovaskulären, kardiopulmonalen, pulmonalen oder renalen Krankheiten**

(30) Priorität: 16.01.2003 DE 10301372; 31.07.2003 DE 10335027
(62) Teilanmeldung aus: 04701918.7
(71) Anmelder: BOEHRINGER INGELHEIM INTERNATIONAL GMBH, D-55216 Ingelheim (DE)
(72) Erfinder: Sendra, José-Maria, 87-50 Bogota (CO); Riedel, Axel Dr., 88437 Maselheim (DE); Leiter, Josef. M. E. Dr., 83067 Holzkirchen (DE); Kauschke, Stefan Dr., 88400 Biberach (DE); Mark, Michael, 88400 Biberach (DE)
(74) Vertreter: Hammann, Heinz

(57) **Zusammenfassung**

Die vorliegende Erfindung betrifft ein Verfahren zur Prophylaxe oder Therapie von kardiovaskulären, kardiopulmonalen, pulmonalen oder renalen Krankheiten durch Besserung der endothelialen Funktion und Erzielung eines Schutzes von Organen, Geweben und Gefäßen bei Indikationen, bei denen eine Blutdruckkontrolle und eine Lipidspiegelkontrolle erforderlich sind, besonders von Personen bei denen Typ 2 Diabetes mellitus diagnostiziert wurde oder Verdacht auf Prädiabetes besteht, zur Prävention von Diabetes und Prädiabetes, oder zur Behandlung des Metabolischen Syndroms und der Insulin Resistenz in Patienten mit normalem Blutdruck. Das Verfahren umfasst die gemeinsame Verabreichung wirksamer Mengen Telmisartan oder eines Polymorphen oder Salzes davon und Simvastatin. Weiters betrifft die Erfindung geeignete pharmazeutische Zusammensetzungen, die Telmisartan oder ein Polymorphes oder Salz davon und Simvastatin enthalten, als Kombinationspräparat für die gleichzeitige, getrennte oder sequenzielle Anwendung bei der Prophylaxe oder Therapie dieser Krankheiten.

## Beschreibung

Die Erfindung betrifft ein Verfahren zur Prophylaxe oder Therapie von kardiovaskulären, kardiopulmonalen, pulmonalen oder renalen Krankheiten, besonders von Personen bei denen Diabetes diagnostiziert wurde oder Verdacht auf Prädiabetes besteht, zur Prävention von Diabetes und Prädiabetes, oder zur Behandlung des Metabolischen Syndroms und der Insulin Resistenz in Patienten mit normalem Blutdruck. Das Verfahren umfasst die gemeinsame Verabreichung wirksamer Mengen des Angiotensin II Rezeptor Antagonisten Telmisartan oder eines Polymorphen oder Salzes davon und Simvastatin an eine behandlungsbedürftige Person. Weiters betrifft die Erfindung geeignete pharmazeutische Zusammensetzungen, die Telmisartan oder ein Polymorphes oder Salz davon und Simvastatin enthalten, als Kombinationspräparat für die gleichzeitige, getrennte oder sequenzielle Anwendung bei der Prophylaxe oder Therapie dieser Krankheiten, sowie die kombinierte Verwendung von Telmisartan oder eines Polymorphen oder Salzes davon und von Simvastatin zur Herstellung einer pharmazeutischen Zusammensetzung zur Prophylaxe oder Therapie dieser Krankheiten.

Angiotensin II (ANG II) spielt eine wichtige Rolle in der Pathophysiologie, insbesondere als das wirkungsstärkste Mittel zur Erhöhung des Blutdrucks beim Menschen. Es ist bekannt, dass ANG II neben seiner blutdrucksteigernden Wirkung ferner wachstumsfördernde Wirkungen aufweist, die zu linker ventrikulärer Hypertrophie, vaskulärer Verdickung, Atherosklerose, Nierenversagen und Schlaganfall beitragen. Auf der anderen Seite übt Bradykinin gefäßerweiternde und gewebeschützende Wirkungen aus. Daher eignen sich ANG II-Antagonisten zur Behandlung von erhöhtem Blutdruck und kongestiver Herzinsuffizienz bei Säugetieren. Beispiele für ANG II Antagonisten sind in EP A 0 502 314, EP A 0 253 310, EP A 0 323 841, EP A 0 324 377, US A 4 355 040 und US A 4 880 804 beschreiben. Beispiele für ANG II Antagonisten sind Candesartan, Eprosartan, Irbesartan, Losartan, Olmesartan, Tasosartan, Valsartan oder Telmisartan. Die antihypertonischen und nierenschützenden Wirkungen von ANG II-Antagonisten sind beispielsweise in den folgenden Veröffentlichungen beschrieben:
- W. Wienen et al.: Antihypertensive and renoprotective effects of telmisartan after long term treatment in hypertensive diabetic (D) rats, 2nd Int. Symposium on Angiotensin II Antagonism, February 15-18, 1999, The Queen Elizabeth II Conference Center, London, UK, Book of Abstracts, Abstract No. 50;
- J. Wagner et al.: Effects of AT1 receptor blockade on blood pressure and the renin angiotensin system in spontaneously hypertensive rats of the stroke prone strain, Clin. Exp. Hypertens., Bd. 20 (1998), S. 205-221; und
- M. Böhm et al.: Angiotensin II receptor blockade in TGR(mREN2)27: effects of renin-angiotensin-system gene expression and cardiovascular functions, J. Hypertens., Bd. 13 (8) (1995), S. 891-899.

Weitere Nierenschutzwirkungen von ANG II-Antagonisten, die bei ersten klinischen Studien festgestellt wurden, sind beispielsweise in folgenden Veröffentlichungen beschrieben:
- S. Andersen et al.: Renoprotective effects of angiotensin II receptor blockade in type 1 diabetic patients with diabetic nephropathy, Kidney Int., Bd. 57 (2) (2000), S. 601-606;
- L.M. Ruilope: Renoprotection and renin-angiotensin system blockade in diabetes mellitus, Am. J. Hypertens., Bd. 10(12 PT 2) Suppl. (1997), S. 325-331; und
- J.F.E. Mann: Valsartan and the kidney: Present and future, J. Cardiovasc. Pharmacol., Bd. 33, Suppl. 1 (1999), S. 37-40.

Ferner sind die Wirkungen von ANG II-Antagonisten auf eine endotheliale Dysfunktion beispielsweise in folgenden Veröffentlichungen beschrieben:
- E.L. Schiffrin et al.: Correction of arterial structure and endothelial dysfunction in human essential hypertension by the angiotensin receptor antagonist losartan, Circulation, Bd. 101(14) (2000), S. 1653-1659;
- R.M. Touyz et al.: Angiotensin II stimulates DNA and protein synthesis in vascular smooth muscle cells from human arteries: role of extracellular signal-regulated kinases, J. Hypertens., Bd. 17(7) (1999), S. 907-916;
- E.L. Schiffrin: Vascular remodelling and endothelial function in hypertensive patients: Effects of antihypertensive therapy, Scand. Cardiovasc. J., Bd. 32, Suppl. 47 (1998) S. 15-21; und
- Prasad: Acute and chronic angiotensin-1 receptor antagonism reverses endothelial dysfunction in atherosclerosis, Circulation, Bd. 101 (2000), S: 2349 ff.. Ferner ist bekannt, dass ANG II-Antagonisten in selektiver Weise den AT1-Rezeptor blockieren, wobei der AT2-Rezeptor, der eine Rolle bei Antiwachstumswirkungen und Geweberegenerationswirkungen zeigt, unberührt bleibt.

EP-A-1 013 273 beschreibt ferner die Verwendung von AT1-Rezeptor-Antagonisten oder AT2-Rezeptor-Modulatoren zur Behandlung von Krankheiten, die mit einer Zunahme der AT1-Rezeptoren im subepithelialen Bereich oder einer Zunahme der AT2-Rezeptoren im Epithel einhergehen, insbesondere zur Behandlung von verschiedenen Lungenkrankheiten.

In anderer Hinsicht wurde festgestellt, dass Hypertonie häufig gleichzeitig mit Hyperlipidämie vorliegt. Beide Erscheinungen gelten als Hauptrisikofaktoren bei der Entwicklung kardiovaskulärer Krankheiten, die häufig zu ungünstigen kardiovaskulären Ereignissen führen. Hohe Blut-Cholesterinspiegel und hohe Blut-Lipidspiegel sind beispielsweise bei beginnender Atherosklerose beteiligt, einem Zustand, der durch unregelmäßig verteilte Lipidablagerungen in der Intima von Arterien, einschließlich Koronar-, Carotis- und peripheren Arterien gekennzeichnet ist. Diese unregelmäßige Lipidverteilung ist auch charakteristisch für koronare Herzleiden, kardiovaskuläre Erkrankungen, deren Schwere und Auftreten auch durch das Vorliegen von Diabetes, durch das Geschlecht der Person, durch Zigarettenrauchen und eine als Nebenerscheinung bei Hypertonie auftretende linke ventrikuläre Hypertrophie beeinflusst wird (Wilson et al., Am. J. Cardiol., Bd. 59(14) (1987), S. 91 G-94G).

Diabetes mellitus Typ 2 ist die Manifestation zweier pathophysiologischer Phänomene, nämlich einer verminderten Insulinsekretion der Beta Zellen des Pankreas und einer Insulinresistenz der Zielorgane Leber, Skelettmuskulatur und Fettgewebe. In der Regel liegt eine komplexe Störung beider Komponenten vor. Die Erkrankung wird als Nüchtern-Hyperglykämie diagnostiziert, d.h. die Blutzuckerkonzentration nach 10-12 Stunden Fasten liegt über dem Grenzwert von 125 mg Glucose pro dl Plasma. Eine gezielte Behandlung des manifesten Typ 2 Diabetes ist durch Verbindungen der Substanzklasse der Thiazolidinedione (Glitazone) möglich. Diese Verbindungen verbessern die Nutzung des zirkulierenden Insulins und führen so zu einer Absenkung der Blutzuckerwerte (Insulinsensitizer). Gleichzeitig werden über Rückkoppelungsmechanismen die erhöhten Insulinspiegel reduziert und damit die Bauchspeicheldrüse entlastet. Insulinsensitizer wie Troglitazone, Rosiglitazone oder Pioglitazone entfalten diese Wirkung durch Bindung an bestimmte nukleäre Rezeptoren, PPAR-gamma genannt (Peroxisomal Proliferator Activated Receptor).

WO 95/06410 offenbart die Verwendung von Angiotensin II Rezeptor Antagonisten zur Behandlung chronischer Entzündungserkrankungen einschließlich systemischer Autoimmunerkrankungen. Als eines von mehreren Beispielen für systemische Autoimmunerkrankungen wird Diabetes genannt. Den Autoimmunerkrankungen zugeordnet wird der Typ 1 Diabetes mellitus, der meist bei Jugendlichen unter 30 Jahren auftritt, wobei es bei entsprechender genetischer Disposition unter dem Einfluss verschiedener Faktoren zu einer Insulitis mit nachfolgender Zerstörung der B-Zellen kommt, sodass der Pankreas nur mehr wenig oder kein Insulin produzieren kann. Typ 2 Diabetes mellitus wird nicht als Autoimmunerkrankung angesehen.

Da zum Zeitpunkt der Diagnose z.B. jeder zweite Typ-2-Diabetes Patient Zeichen einer koronaren Herzerkrankung aufweist, werden die Ursachen von Diabetes zunehmend in einer komplexen Stoffwechselstörung vermutet, die durch eine Reihe von Risikofaktoren wie gestörte Glukose-Toleranz, erhöhten Nüchternblutzucker, Insulinresistenz, Bluthochdruck, Dyslipidämie, oder Stammfettsucht angezeigt werden kann. Besonders ausgeprägt ist die Prävalenz einer Insulinresistenz bei Patienten mit Hypertriglyzeridämie und niedrigem HDL-Cholesterin. Man spricht vom Prä-Typ 2-Diabetes, Metabolischen Syndrom, Syndrom X, oder Insulin-Resistenz Syndrom. In einer ersten Phase bedingt eine verminderte Insulinantwort der Zielorgane eine Steigerung der pankreatischen Insulinsekretion, um den Blutzuckerspiegel im Normbereich zu halten. Nach mehreren Jahren überhöhter bzw. steigender Insulinproduktion kommt es dazu, dass die Insulinausschüttung durch die Beta-Zellen des Pankreas nicht weiter gesteigert werden kann. Damit beginnt die Phase der gestörten Glukosetoleranz. Der Organismus kann Glukose-Spitzenwerte nicht mehr rasch genug auffangen. Bleibt der Nüchternblutzucker schliesslich anhaltend hoch wird der Diabetes manifest.

Angina pectoris, ein Zustand, der durch starke Konstriktionsschmerzen in der Brust gekennzeichnet ist, die häufig von der Herzgegend in die linke Schulter und hinunter zum linken Arm ausstrahlen, wird häufig durch eine Kombinationstherapie von β-Blocker, Nitrat oder Calciumkanal-Blocker zusammen mit einem Lipidsenker behandelt. Häufig ist Angina pectoris auf eine Herzischämie zurückzuführen und wird üblicherweise durch eine Koronarkrankheit verursacht. Bei Behandlung durch chirurgische Maßnahmen kommt es bei Angina pectoris-Patienten häufig zu Komplikationen, wie Restenose, die sich entweder als eine kurzzeitige proliferative Reaktion auf das durch die Angioplastik herbeigeführte Trauma oder als Langzeitprogression des arteriosklerotischen Vorgangs sowohl in transplantierten Gefäßen als auch in Angioplastik-Segmenten bemerkbar machen.
Einige Therapiemöglichkeiten zur Senkung von Lipiden und Cholesterin beruhen auf der Hemmung der Aktivität des Enzyms 3-Hydroxy-3-methylglutaryl-Coenzym A-Reduktase (HMG-CoA-Reduktase), das die Umwandlung von 3-Hydroxy-3-methylglutaryl-Coenzym A zu Mevalonat katalysiert, eine frühe Stufe im biosynthetischen Cholesterin-Stoffwechselweg. Bekannte Inhibitoren der HMG-CoA-Reduktase sind beispielsweise Verbindungen, die sich von einem Pilzmetaboliten ableiten und deren eingeführte Bezeichnungen mit "statin" enden, z. B. Pravastatin, Lovastatin, Fluvastatin, Simvastatin oder Atorvastatin.
Simvastatin ist als wirkungsstarker Inhibitor des Enzyms 3-Hydroxy-3-methylglutaryl-Coenzym A-Reduktase (HMG-CoA-Reduktase) und als Inhibitor der Cholesterin-Biosynthese bekannt, dessen Wirkung über eine Senkung von Lipoprotein-Cholesterin niedriger Dichte (LDL-C) verläuft. Diese Wirkungen begründen die Attraktivität dieses Moleküls bei der Behandlung einer kombinierten Hyperlipidämie, einer üblichen atherogenen Störung in der klinischen Praxis, und somit auch bei der Prophylaxe der Atherom-Progression.
Untersuchungen haben ferner ergeben, dass eine Senkung der LDL-C-Spiegel einen Schutz gegen koronare Herzkrankheiten bietet (vergl. z. B. "Scandinavian Simvastatin Survival Study" oder 4S-Studie, veröffentlicht in The Lancet, Bd. 344 (1994), S. 1383-1389, oder die Studie "Prevention of coronary heart disease with privastatin in men with hypercholesterolemia", veröffentlicht von Shepherd et al., in The New England Journal of Medicine, Bd. 333 (1995), S. 1301-1307).
Weitere Studien werden durchgeführt, um das Schutzvermögen von Statinen gegen das Auftreten von Herzanfällen, Schlaganfällen und koronaren Herzkrankheiten bei nicht-insulinabhängigen Diabetikern zu bestimmen; "Collaborative Atorvastatin Diabetes Study" oder die CARDS-Studie "Atorvastatin Versus Revascularisation Treatment", die AVERT-Studie und die "Anglo-Scandinavian Cardiac Outcomes trial" oder ASCOT-Studie.

Da, wie vorstehend erwähnt, Hochdruck häufig zusammen mit Hyperlipidämie oder Anzeichen von Typ 2 Diabetes auftritt und da diese Erscheinungen Hauptrisikofaktoren bei der Entwicklung von kardiovaskulären Krankheiten, die häufig zu nachteiligen kardiovaskulären Ereignissen führen, darstellen, wäre es für die Patienten von Vorteil, über eine einzige Therapie zu verfügen, die diesen Zuständen vorbeugt oder diese behandelt.
Außerdem wäre es von Vorteil, wenn die Kombinationstherapie auch eine Verbesserung der Prophylaxe oder Therapie von kardiopulmonalen, pulmonalen oder renalen Krankheiten, für die festgestellt wurde, dass ANG II-Antagonisten wirksam sind, bewirken würde.
Ziel der vorliegenden Erfindung ist es, Arzneimittel bereitzustellen, die sich sowohl zur Behandlung von Bluthochdruck als auch zur Behandlung einer Hyperlipidämie eignen, die Behandlung des Metabolischen Syndroms und der Insulin Resistenz ermöglichen und gleichzeitig zur Behandlung eines manifesten Typ 2 Diabetes als auch zur Behandlung erster Anzeichen der komplexen Stoffwechselstörung des Prädiabetes und damit zur Prävention eines Typ 2 Diabetes mellitus verwendet werden können.

Es wurden bereits Kombinationsbehandlungen und entsprechende Zusammensetzungen, die HMG-CoA-Reduktase-Inhibitoren und ANG II-Antagonisten enthalten, vorgeschlagen.
- WO-95/26188 beschreibt ein Verfahren zur Behandlung von Atherosklerose und zur Verringerung von Cholesterin, wobei man sich eines HMG-CoA-Reduktase-Inhibitors und eines ANG II-Antagonisten bedient. Pravastatin, Simvastatin und Lovastatin werden als mögliche HMG-CoA-Reduktase-Inhibitoren, die für den Einsatz in Frage kommen, erwähnt. Losartan wird als möglicherweise einzusetzender ANG II-Antagonist erwähnt.
- WO 97/37688 beschreibt die kombinierte Verwendung von HMG-CoA-Reduktase-Inhibitoren und ANG II-Antagonisten zur Behandlung zahlreicher Zustände, darunter Hypertonie und Atherosklerose. Pravastatin, Simvastatin, Lovastatin and Fluvastatin werden als mögliche HMG-CoA-Reduktase-Inhibitoren, die verwendet werden können, erwähnt.
- WO 99/11260 beschreibt die kombinierte Verwendung eines speziellen HMG-CoA-Reduktase-Inhibitors und von ANG II-Antagonisten zur Senkung des Blutdrucks und der Lipidspiegel sowie zur Behandlung von Angina pectoris und Atherosklerose bei Säugetieren. Beim speziellen HMG-CoA-Reduktase-Inhibitor handelt es sich um Atorvastatin. Losartan, Irbesartan und Valsartan werden als mögliche ANG II-Antagonisten, die vorzugsweise zu verwenden sind, erwähnt. Weitere erwähnte ANG II-Antagonisten sind Candesartan und Eprosartan.
- WO 00/45818 beschreibt die kombinierte Verwendung eines HMG-CoA-Reduktase-Inhibitors und eines ANG II-Antagonisten zur Besserung von diabetischer Neuropathie und insbesondere zur Verbesserung der Nervenleitungsgeschwindigkeit und der Nervendurchblutung bei an Diabetes leidenden Patienten. Bei den erwähnten Beispielen für mögliche Kombinationen handelt es sich um Kombinationen aus den Statinen Pravastatin, Simvastatin, Cerivastatin, Fluvastatin, Atorvastatin und Statin (E) zusammen mit den ANG II-Antagonisten Losartan, Irbesartan, Valsartan und Candesartan, wobei Candesartan bevorzugt wird.
- WO 01/15674 beschreibt die Kombination eines Inhibitors des Renin-Angiotensin-Systems zusammen mit einem weiteren antihypertonischen, cholesterinsenkenden Mittel, einem Diuretikum oder Aspirin zur Verhinderung kardiovaskulärer Ereignisse, wie Schlaganfall, kongestiver Herzinsuffizienz, kardiovaskulärem Tod, Myokardinfarkt, Verschlechterung von Angina, Herzstillstand, Revaskularisationsvorgänge, Diabetes und diabetische Komplikationen. Als Beispiele für mögliche Kombinationen sind die Kombinationen von Inhibitoren des Angiotensin-Converting-Enzyms (ACE), d. h. Verbindungen, deren eingeführte Namen die Endung "-pril" tragen, wie Captopril, Imidapril, Ramipril und dergl., mit den Cholesterinspiegelsenkern Lovastatin, Pravastatin, Simvastatin oder Fluvastatin erwähnt.

Im Rahmen der vorliegenden Erfindung wurde nun überraschend gefunden, dass der Angiotensin II Rezeptor Antagonist Telmisartan und seine Salze neben der bekannten, den Blutdruck senkenden Wirkung, in einem zellulären System auch die Expression von Genen zu steigern vermag, von deren Transkription bekannt ist, dass sie durch den PPARgamma Rezeptor reguliert wird. Um vergleichbare Bedingungen zu gewährleisten wird dieser Effekt im Rahmen der vorliegenden Erfindung mit Hilfe einer stabil transormierten Zelllinie (vgl. Beispiel 2) beobachtet und quantifiziert. Dabei handelt es sich um CHO-Zellen, die das Ergebnis einer Transformation mit zwei Genkonstrukten darstellen. Das erste dieser Konstrukte kodiert für das Luciferase Gen aus *Photinus pyralis* (de Wet JR, Mol Cell Biol (1987) 7:725) unter der Kontrolle eines synthetischen Promotors mit einer fünffachen Wiederholung einer Hefe Gal4-Bindungsstelle (vgl. Genbank-Sequenz AF058756). Das zweite Konstrukt kodiert für ein Fusionsprotein bestehend aus der Ligandenbindungsdomäne des humanen PPARgamma2 Transkriprionsfaktors (vgl Genbank-Sequenz U79012) sowie der Hefe GAL4 DNA Bindungsdomäne (Aminosäuren 1-147; Sadowski I, Nucleic Acids Res (1989) 17:7539).

Die Induktion der Transkription von PPARgamma regulierten Genen ist von den als Antidiabetika verwendeten Thiazolidinedionen (z.B. Rosiglitazone) bekannt und wird durch deren Bindung an den PPARgamma Rezeptor und dessen Aktivierung bewirkt. Im Rahmen des hier angewandten Testsystems kann diese Wirkung als induzierte Luciferase Aktivität der transformierten Zellinie quantifiziert werden. Dieselbe Induktion einer Luciferase Aktivität erfolgt bei Telmisartan wider Erwarten nicht durch Bindung des Wirkstoffs an den Rezeptor PPARgamma. Eine Bindung von Telmisartan an den PPARgamma Rezeptor kann in verschiedenen Testsystemen nicht nachgewiesen werden. Es wird daher vermutet, dass die Erhöhung der Affinität von Cofaktorproteinen für PPARgamma durch den Angiotensin II Rezeptor Antagonisten Telmisartan auch dann zur Rekrutierung der Cofaktorproteine führt, wenn hochaffine synthetische PPARgamma-Liganden nicht vorliegen. Dies bewirkt dann eine durch diese Cofaktoren vermittelte Aktivierung der Transkription von Genen, die durch den PPARgamma Rezeptor reguliert werden. Da nun die Induktion dieser Gene für die anti-diabetische Wirkung der Thiazolidinedione verantwortlich ist, kann davon ausgegangen werden, dass die Induktion derselben Gene durch Telmisartan eine vergleichbare anti-diabetische Wirkung entfaltet. Somit eignet sich Telmisartan nicht nur zur Behandlung von Bluthochdruck sondern auch zur Behandlung und Prävention von Typ 2 Diabetes mellitus. Darin eingeschlossen ist die Behandlung und Prävention von Metabolischem Syndrom, Syndrom X, bzw. Insulin-Resistenz Syndrom.

Die Entdeckung dieser neuen therapeutischen Wirkung von Telmisartan und dessen Salzen bedeutet, dass sie verwendet werden können zur Herstellung eines Arzneimittels für die Behandlung von Personen oder Säugetieren bei denen die Prophylaxe oder Therapie von kardiovaskulären, kardiopulmonalen, pulmonalen oder renalen Krankheiten angezeigt ist, vor allem wenn Diabetes Mellitus Typ 2 diagnostiziert wurde oder Verdacht auf Prädiabetes besteht, oder wenn trotz normalem Blutdruck die übrigen Daten auf das Vorliegen des Metabolischen Syndroms oder einer Insulin Resistenz schließen lassen. Sie sind also zur Behandlung und Prävention von Typ2-Diabetes und Prä-Typ2-Diabetes geeignet. Darin eingeschlossen ist die Behandlung und Prävention von Metabolischem Syndrom, Syndrom X, bzw. Insulin-Resistenz Syndrom. Von besonderer Bedeutung ist die Behandlung von Personen bei denen die Prophylaxe oder Therapie von Hypertonie kombiniert mit Hyperlipidämie oder Atherosklerose angezeigt ist, oder die Behandlung von Asthma, Bronchitis oder interstitiellen Lungenkrankheiten.

Diabetes mellitus Typ 2 manifestiert sich in einem Nüchternblutzuckerwert, der 125 mg Glucose pro dl Plasma überschreitet, wobei die Bestimmung von Glucosewerten im Blut ein Standardverfahren in der medizinischen Routineanalytik darstellt. Wird eine Glukose Toleranz Test durchgeführt so liegt 2 Stunden nach nüchterner Einnahme von 75 g Glucose der Blutzuckerwert eines Diabetikers über 200mg Glucose pro dl Plasma. Bei einem Glukose Toleranz Test werden der zu untersuchenden Person nach 10-12 stündigem Fasten 75 g Glucose oral verabreicht und der Blutzuckerwert wird unmittelbar vor Einnahme sowie 1 bzw. 2 Stunden nach Einnahme der Glucose ermittelt. Bei einem Gesunden liegt der Blutzuckerwert vor Einnahme zwischen 60 und 110 mg pro dl Plasma, eine Stunde nach Einnahme unter 200 mg pro dl und nach 2 Stunden unter 140 mg pro dl. Liegt der Wert nach 2 Stunden zwischen 140 und 200 mg so spricht man auch von einer gestörten Glukosetoleranz.

Ein besonders starkes Indiz für das Vorliegen eines Prädiabetes ist, wenn eine Insulinresistenz nachgewiesen werden kann. So kann es sein, dass eine Person zur Aufrechterhaltung der Glukosehomöostase die 2-3fache Menge an Insulin benötigt als eine andere, ohne dass dies eine direkte pathologischen Bedeutung hätte. Als die aussagekräftigste Methode zur Bestimmung der Insulinresistenz gilt der euglykämisch-hyperinsulinämische Clamp-Test. Das Verhältnis Insulin zu Glukose wird im Rahmen einer kombinierten Insulin-Glukose-Infusionstechnik bestimmt. Von einer Insulinresistenz spricht man, wenn die Glucoseaufnahme unterhalb der 25. Perzentile der untersuchten Hintergrundspopulation liegt (WHO Definition). Etwas weniger aufwendig als die Clamp-Untersuchung sind sogenannte Minimalmodelle bei denen während eines intravenösen Glukosetoleranz-Tests in zeitlich festgelegten Abständen Insulin- und Glukosekonzentrationen im Blut gemessen werden und hieraus die Insulinresistenz berechnet wird. Ein weiteres Bestimmungsverfahren ist das mathematische HOMA-Modell. Die Insulinresistenz wird über die Nüchtern-Plasma-Glukose und die Nüchtern-Insulin-Konzentration berechnet. Im Rahmen dieser Methode ist es nicht möglich zwischen hepatischer und peripherer Insulinresistenz zu unterscheiden. Für eine Bewertung der Insulinresistenz in der täglichen Praxis sind diese Verfahren wenig geeignet. Im klinischen Alltag werden zur Einschätzung der Insulinresistenz in der Regel andere Parameter herangezogen. Bevorzugt wird dafür die Triglyzerid-Konzentrationen des Patienten herangezogen werden, da erhöhte Triglyzerid-Spiegel signifikant mit dem Vorliegen einer Insulinresistenz korrelieren.

So liegt ein Verdacht auf Prädiabetes vor, wenn der Nüchternblutzuckerwert über dem maximalen Normalwert von 110 mg Glucose pro dl Plasma liegt aber den für Diabetes relevanten Grenzwert von 125 mg Glucose pro dl Plasma nicht überschreitet. Ein anderes Indiz für Prädiabetes ist eine gestörte Glukose Toleranz, d.h. ein Blutzuckerwert von 140-200mg Glucose pro dl Plasma 2 Stunden nach nüchterner Einnahme von 75 g Glucose im Rahmen eines Glukose Toleranz Tests.

Auch ein Blutwert für Triglyzeride von mehr als 150 mg/dl lässt das Vorliegen von Prädiabetes vermuten. Dieser Verdacht wird durch niedrige Blutwerte für HDL-Cholesterin bestärkt. Bei Frauen sind Werte unter 40 mg pro dl Plasma, bei Männern Werte unter 50 mg pro dl Plasma als zu niedrig zu werten. Die Bestimmung von Triglyzeriden und HDL-Cholesterin im Blut gehört ebenfalls zu den Standardverfahren in der medizinischen Analytik und wird z.B. in: Thomas L (Hrsg.): Labor und Diagnose", TH-Books Verlagsgesellschaft mbH, Frankfurt/Main, 2000 beschrieben. Weiter bestärkt wird ein Verdacht auf Prädiabetes, wenn die Nüchternblutzuckerwerte gleichzeitig 110 mg Glucose pro dl Plasma überschreiten. Liegen die gemessenen Blutwerte im Bereich der Grenzwerte, so kann als zusätzliche Entscheidungshilfe das Verhältnis des Taillienumfangs zum Hüftumfang herangezogen werden. Überschreitet dieses Verhältnis bei Frauen den Wert 0,8 bzw. bei Männern den Wert 1 so ist eine Behandlung angezeigt.

Besonders angezeigt für die Behandlung von Diabetes bzw. vermuteter Prädiabetes ist Telmisartan dann, wenn auch eine Hypertonie behandelt werden muss. Dies ist der Fall, wenn der systolische Blutdruck einen Wert von 140 mm Hg und der diastolische Blutdruck einen Wert von 90 mm Hg übersteigt. Leidet ein Patient bereits an manifester Diabetes wird heute empfohlen den systolischen Blutdruck auf einen Wert unter 130 mm Hg und den diastolische Blutdruck auf einen Wert unter 80 mm Hg zu senken. Um diese Werte zu erreichen kann von Fall zu Fall die Kombination des Angiotensin II Rezeptor Antagonisten mit einem Diuretikum oder einem Calcium Antagonisten angezeigt sein. Der Begriff "Diuretikum" umfasst Thiazide bzw. Thiazidanaloga wie Hydrochlorothiazide (HCTZ), Clopamide, Xipamide oder Chlorthalidone, Aldosteron-Antagonisten wie Spironolacton oder Eplerenone als auch andere Diuretika, die sich zur Behandlung von erhöhtem Blutdruck eignen wie Furosemide und Piretanide, und deren Kombinationen mit Amiloride und Triamteren.

Die vorliegende Erfindung bedeutet, dass für Personen, die wegen eines erhöhten Blutdrucks behandelt werden, der Angiotensin I Rezeptor AntagonistTelmisartan immer dann indiziert ist, wenn der Entwicklung eines Prädiabetes vorgebeugt bzw. ein manifester Diabetes behandelt werden soll.

In nur 10 Prozent aller Fälle von erhöhtem Blutdruck (sekundäre Hypertonie) ist eine identifizierbare Ursache feststellbar, wie z.B. eine Nierenerkrankung. Durch Behandlung und Beseitigung der Ursache lässt sich diese sekundäre Hypertonie in der Regel beheben. In fast 90 Prozent aller Fälle handelt es sich aber um eine primäre Hypertonie, deren genaue Ursache nicht bekannt und eine direkte Heilung somit nicht möglich ist. Die negativen Auswirkungen des erhöhten Blutdrucks können durch Veränderung der Lebensgewohnheiten und eine richtige Behandlung vermindert werden. Das Zusammenspiel verschiedener bzw. das gemeinsame Auftreten einzelner Risikofaktoren scheint den Bluthochdruck zu verursachen. Vor allem das Zusammentreffen von Bluthochdruck mit Störungen des Fett- und Zuckerstoffwechsels wird vermehrt beobachtet. Diese Störungen bleiben zunächst oft unbemerkt, können aber anhand erhöhter Blutwerte von Triglyzeriden und Glucose sowie erniedrigten Blutwerten von HDL-Cholesterin erkannt werden. In etwas fortgeschrittenerem Stadium sind sie zusätzlich an einer langsam zunehmenden Fettleibigkeit zu erkennen. Erklären lassen sich diese Störungen durch zunehmende Insulin Resistenz. Je weniger wirksam das Insulin ist, desto mehr gerät der Fett- und Zuckerstoffwechsel durcheinander. Die Kombination all dieser Störungen erhöht letztlich die Wahrscheinlichkeit, an der Zuckerkrankheit Diabetes zu leiden und vorzeitig an einer Herz- oder Gefäßerkrankung zu leiden oder zu sterben.

Da die primäre oder essentielle Hypertonie eine multifaktorielle Erkrankung darstellt, erscheint es unwahrscheinlich, dass Insulinresistenz oder Hyperinsulinämie die alleinige Ursache eines Bluthochdrucks bedeuten. Eine Reihe von Beobachtungen deutet jedoch darauf hin, dass Defekte im Insulinmetabolismus hypertensiv wirken und somit für Bluthochdruck prädisponieren. In diesem Zusammenhang kann von einer hypertensiven Insulinresistenz gesprochen werden. So kann bei etwa 50% der normalgewichtigen Hypertoniker und bei normotensiven Verwandten ersten Grades bereits das Vorhandensein einer Insulinresistenz nachgewiesen werden. Bei adipösen Patienten findet sich nicht nur ein höheres Ausmaß der Insulinresistenz, sondern auch eine stärkere Korrelation zwischen Hypertonie und Hyperinsulinämie als bei schlanken Hypertonikern.

Schätzungen gehen davon aus, dass etwa ein Drittel der Erwachsenen in den Regionen der Erde, die mit Nahrung überversorgt sind, vom Zusammentreffen eines erhöhten Blutdrucks mit Störungen des Fett- und Zuckerstoffwechsels betroffen sind und dass diese Zahl noch zunehmen wird. Daraus resultiert ein Bedarf an Arzneimitteln, die in der Lage sind dazu beizutragen, das Fortschreiten der genannten Stoffwechselstörungen in einer möglichst frühen Phase zu verlangsamen oder zu stoppen und gleichzeitig die gesundheitsschädlichen Auswirkungen eines erhöhten Blutdrucks zu vermeiden.

Die vorliegende Erfindung offenbart nun ein Arzneimittel, das sowohl zur gleichzeitigen Behandlung von Hypertonie und Hyperlipidämie als auch zur Behandlung eines manifesten Typ 2 Diabetes bzw. erster Anzeichen der komplexen Stoffwechselstörung des Prädiabetes verwendet werden kann. Die neue Wirkstoffkombination ist besonders geeignet zur Behandlung und Prävention der oben genannten hypertensiven Insulinresistenz, welche eine unzureichende Nutzung des im Blutkreislauf zirkulierenden Insulins darstellt, verbunden mit einer dadurch bedingten Erhöhung des Blutdrucks. Somit umfasst die vorliegende Erfindung auch die Diabetes Prävention in Patienten, die wegen erhöhtem Blutdruck und Hyperlipidämie behandelt werden. Wird die Kombination von Telmisartan und Simvastatin sofort dann zur Kontrolle von Blutdruck, Hyperlipidämie oder hypertensiver Insulinresistenz verwendet, wenn eines der genannten Anzeichen auf Prädiabetes vorliegt, kann dadurch das Auftreten eines manifesten Typ 2 Diabetes verzögert oder vermieden werden.

Telmisartan und seine geeigneten Salze zeigen also
- keine *in vitro* Bindung an die Ligandenbindungsdomäne eines humanen PPARgamma Rezeptors, führen aber zur
- Induktion einer Luciferase Aktivität, wenn sie zum Kulturmedium einer stabil transformierten PPARgamma-Reporterzelllinie zugegeben werden, welche
   a) ein Fusionsprotein bestehend aus der Ligandenbindungsdomäne des humanen PPARgamma Transkriprionsfaktors sowie der Hefe GAL4 DNA Bindungsdomäne exprimiert und
   b) ein Luciferase Gen unter der Kontrolle einer fünffach wiederholten Hefe Gal4-Bindungsstelle enthält.

Die Herstellung einer solchen PPARgamma-Reporterzelllinie wird in Beispiel 2 beschrieben.

Eine *in vitro* Bindung an die Ligandenbindungsdomäne des humanen PPARgamma2 Rezeptors liegt dann nicht vor, wenn sie in einem AlphaScreen (Ullmann EF et al, Proc Natl Acad Sci USA (1994) 91:5426-5430) nicht nachgewiesen werden kann. Anstelle eines Alpha Screens kann auch ein SPA-Assay (Mukherjee R et al., J Steroid Biochem Mol Biol (2002) 81:217-225) oder eine NMR-Untersuchung (Johnson BA et al., J Mol Biol (2000) 298:187-194) durchgeführt werden. In der Regel kann mit keinerm dieser Verfahren eine Bindung an den Rezeptor nachgewiesen werden.

Erscheint die kombinierte Anwendung eines Angiotensin II Rezeptor Blockers mit ein oder mehreren anderen therapeutischen Wirkstoffen sinnvoll oder notwendig, stellt Telmisartan einen bevorzugten Angiotensin II Rezeptor Blocker dar, weil er in einem einzigen Wirkstoff eine Blutdruck-senkende mit einer antidiabetischen Wirkung kombiniert bzw. zur Prävention von Diabetes beiträgt. Aus diesem Grund bedeutet eine vorformulierte Wirkstoffkombinationen von Telmisartan mit dem HMG-Co A Reduktase Inhibitor Simvastatin eine wegweisende Weiterentwicklung in der Therapie von kardiovaskulären, kardiopulmonalen, pulmonalen oder renalen Krankheiten, vor allem bei gleichzeitig notwendiger Behandlung von Hypertonie, Hyperlipidämie, Prädiabetes oder manifestem Type 2 Diabetes, Osteoporose oder Alzheimer sowie der Diabetes Prävention.

Es wird beobachtet, dass durch eine gemeinsame Verabreichung einer wirksamen Menge Telmisartan oder eines Polymorphen oder Salzes davon, mit einer wirksamen Menge Simvastatin, überraschende Vorteile bei der Prophylaxe oder Therapie von kardiovaskulären, kardiopulmonalen, pulmonalen oder renalen Krankheiten bei behandlungsbedürftigen Personen mit hohem Wirkungsgrad erzielt werden können, unabhängig von der bekannten blutdrucksenkenden Aktivität des Wirkstoffs Telmisartan und unabhängig von der antihyperlipidämischen Aktivität des Wirkstoffs Simvastatin, verglichen mit der Verabreichung des ANG II-Antagonisten oder des HMG-CoA-Reduktase-Inhibitors allein. So wird z.B. eine Kontrolle der Expression der Matrix-Metalloproteinase MMP-9 möglich, die bei chronischen Entzündungen der Atemwege oder bei Typ 2 Diabetes verstärkt expremiert wird. Auch kann erhöhten Plasmawerten des Entzündungsfördernden Cytokins CD40L entgegengewirkt werden. Erhöhte Plasmawertes von CD40L sind ein bekannter Risikofaktor für kardiovaskuläre Erkrankungen.

Ferner wird beobachtet, dass die Prophylaxe oder Therapie die endotheliale Funktion bessert und einen Schutz von Organen, Geweben und Gefäßen bei Krankheiten, bei denen die Notwendigkeit zur Kontrolle sowohl des Blutdrucks als auch der Lipidspiegel besteht, bewirkt. So kann die Elastizität der Arterien verbessert und in der Haut eine verstärkte Produktion von NO, einem Marker der endothelialen Funktion, erreicht werden.
Ferner wird beobachtet, dass die Prophylaxe oder Therapie in folgenden Situationen besonders wirksam ist:
Indikationen (A), die in positiver Weise durch eine Hemmung von durch den AT1-Rezeptor vermittelten Wirkungen und eine Aufrechterhaltung von durch den AT2-Rezeptor vermittelten Wirkungen von Angiotensin II (ANG II) und durch eine Hemmung der HMG-CoA-Reduktase-Wirkungen beeinflusst werden können, wodurch somit auch die durch Bradykinin vermittelten Wirkungen verstärkt und antihyperlipidämische Wirkungen erzielt werden können; oder
Indikationen (B), die mit einer Zunahme der AT1-Rezeptoren im subepithelialen Bereich oder einer Zunahme der AT2-Rezeptoren im Epithel einhergehen. Geeignete Indikationen (A) werden unter folgenden Indikationen ausgewählt: Behandlung von kombinierter Hypertonie und Hyperlipidämie;
   vermindertes Auftreten von Schlaganfällen, akuten Myokardinfarkten oder kardiovaskulären Todesfällen, insbesondere bei Personen mit einem erhöhten Risiko von nachteiligen kardiovaskulären Ereignissen oder Schlaganfällen;
   Bereitstellung einer Nierenschutzwirkung, z. B. bei Niereninsuffizienz oder diabetischer Nephropathie;
   Verhinderung von linker ventrikulärer Hypertrophie, vaskulärer Verdickung, z. B. Verhinderung der Verdickung von Blutgefäßwänden nach Gefäßoperationen, Besserung der Überlebenschancen nach Herztransplantationen, Verhinderung von arterieller Restenose nach Angioplastik, Prophylaxe oder Therapie von atherogenen Störungen, wie Atherosklerose, Schutz gegen Koronararterien-krankheiten, Verhinderung der Atherom-Progression und Prophylaxe von diabetischer Angiopathie;
   Senkung von Cholesterin, Senkung von Plasma-Fibrinogen und der Plasma-Viskosität, Hemmung der Proliferation von glatten Muskelzellen, Verringerung der Fähigkeit von Makrophagen zur Oxidation von LDL, Schutz von Herzmuskelzellen gegen hypoxische Schädigungen und Senkung des Plasminogenaktivator-Inhibitors-1 (PAI-1);
   Prophylaxe oder Therapie von ischämischen peripheren Kreislaufstörungen und Myokardischämie (Angina); und
   Verhinderung der Progression von Herzinsuffizienz nach Myokardinfarkten. Geeignete Indikationen (B) werden unter folgenden Indikationen ausgewählt: obstruktive Luftwegerkrankungen, chronische obstruktive Lungenerkrankungen, wie Bronchitis oder chronische Bronchitis, Emphyseme, beispielsweise aufgrund von Asthma, zystische Fibrose, interstitielle Lungenkrankheiten, Lungenkrebs, pulmonale Gefäßerkrankungen und erhöhter Widerstand gegen den Luftstrom bei forcierter Ausatmung;
   Atemnotsyndrom bei Erwachsenen (ARDS), Verringerung der proliferativen Kapazität des Epithels bei Lungen- und Brustkrebs, Behandlung von Sepsissyndromen, Lungenschädigungen, wie Lungenentzündung, Absaugen von Mageninhalt, Brustkorbtrauma, Schock, Verbrennungen, Fettembolien, kardiopulmonaler Bypass, O₂-Toxizität, hämorrhagische Pankreatitis, interstitielle und bronchoalveoläre Entzündungen, vor allem, wenn sie mit einer erhöhten Expression von Matrix-Metalloproteinase wie MMP-9 einhergehen, Proliferation von epithelialen und
interstitiellen Zellen, Kollagenanreicherung und Fibrose.
   Somit stellt die vorliegende Erfindung ein Verfahren zur Prophylaxe oder Therapie von Hypertonie und Hyperlipidämie bereit, vor allem bei einem Säugetier, bei dem Diabetes diagnostiziert wurde oder Verdacht auf Prädiabetes besteht, wobei das Verfahren die gemeinsame Verabreichung einer wirksamen Menge des HMG-CoA-Reduktase-Inhibitors Simvastatin zusammen mit einer wirksamen Menge des ANG II - Antagonisten Telmisartan oder eines Polymorphen oder Salzes davon umfasst. Ferner betrifft die vorliegende Erfindung die kombinierte Verwendung von Simvastatin und Telmisartan oder eines Polymorphen oder Salzes davon bei der Herstellung einer pharmazeutischen Zusammensetzung zur Prophylaxe oder Therapie von Hypertonie in Kombination mit Hyperlipidämie, vor allem wenn Diabetes diagnostiziert wurde oder Verdacht auf Prädiabetes besteht.
   Somit beruht die vorteilhafte Wirkung der erfindungsgemäßen Verfahren vorwiegend auf der Schutzwirkung der Kombinationsbehandlung für Organe, Gewebe und Gefäße sowie der Präventionswirkung in bezug auf Diabetes.
   Die vorerwähnten unerwarteten Vorteile können auf eine wirksamere Blockade der durch den AT1-Rezeptor vermittelten Wirkungen von ANG II, auf die durch den AT2-Rezeptor vermittelte Wirkung von ANG II, die durch diesen spezifischen ANG II-Antagonisten unbeeinflusst bleibt, zusammen mit einem Anstieg der durch Bradykinin vermittelten Wirkungen, auf die PPARgamma ähnliche Transkriptionsaktivierung und auf die Erzielung einer antihyperlipidämischen Wirkung durch Simvastatin zurückzuführen sein.
   Beispielweise wird beobachtet, dass die gemeinsame Verabreichung des spezifischen ANG II-Antagonisten Telmisartan oder eines Polymorphen oder Salzes davon mit dem spezifischen HMG-CoA-Reduktase-Inhibitor Simvastatin eine signifikante Verhinderung von kardiovaskulären Todesfällen und der Gesamtmortalität, insbesondere in Bezug auf das Auftreten von Schlaganfällen und akuten Myokardinfarkten bewirkt, verglichen mit der Verabreichung eines dieser Wirkstoffe allein.
   Daher besteht ein bevorzugtes erfindungsgemäßes Verfahren darin, das Auftreten von Schlaganfällen und akuten Myokardinfarkten bei Menschen oder nicht-humanen Säugetieren, die einer Behandlung bedürfen, insbesondere bei Personen mit manifestem Typ 2 Diabetes oder Verdacht auf Prädiabetes oder mit einem erhöhten Risiko von nachteiligen kardiovaskulären Ereignissen oder Schlaganfällen, zu verringern, indem man Telmisartan oder ein Polymorphes oder Salz davon gemeinsam mit Simvastatin verabreicht.

Ferner wird beobachtet, dass die Kombinationsbehandlung und die entsprechenden Zusammensetzungen, die spezifisch eine Menge des HMG-CoA-Reduktase-Inhibitors Simvastatin zusammen mit einer Menge des ANG II-Antagonisten Telmisartan oder eines Polymorphen oder Salzes davon enthalten, eine hohe Aktivität bei der Regulierung des Blutdrucks und bei der Lipidregulierung bei einem Säugetier bewirken. Es wird erwartet, dass die synergistische Wirkung, die durch diese spezielle Kombination erreicht wird, in überraschender Weise der Wirkung entsprechender herkömmlicher Kombinationen überlegen ist. Unter einer synergistischen Kombination zur Blutdruckregulierung und Lipidregulierung ist zu verstehen, dass sie eine Menge an Simvastatin und eine Menge an Telmisartan oder eines Polymorphen oder Salzes dieses Wirkstoffes enthält, wobei die Menge des einzelnen Wirkstoffes allein nicht ausreicht, die therapeutische Wirkung zu erzielen, die durch Verabreichung der Kombination der Mittel erreicht wird, und wobei die kombinierten Wirkungen der Mengen der therapeutischen Mittel größer sind als die Summe der therapeutischen Wirkungen, die mit den Mengen der einzelnen therapeutischen Mittel erreichbar ist.

Ein weiterer Gegenstand der vorliegenden Erfindung sind pharmazeutische Zusammensetzungen umfassend Telmisartan oder eines seiner Salze in Kombination mit Simvastatin sowie ihre Herstellung. Sie dienen der Behandlung des humanen oder nicht-humanen Säugetierkörpers zur Prophylaxe oder Therapie der vorerwähnten Krankheiten oder Indikationen, wobei sie Telmisartan und Simvastatin gegebenenfalls zusammen mit pharmazeutisch verträglichen Verdünnungsmitteln und/oder Trägerstoffen umfassen, und zwar in Form eines Kombinationspräparats zur gleichzeitigen, getrennten oder aufeinanderfolgenden Verwendung bei der Prophylaxe oder Therapie dieser Krankheiten oder Indikationen.

Diese Wirkstoffkombinationen werden in der Regel mit einem oder mehreren Formulierungshilfsstoffe wie Mannitol, Sorbitol, Xylit, Saccharose, Calciumcarbonat, Calciumphosphat, Lactose, Croscarmellose Natriumsalz (Cellulose carboxymethylether Natriumsalz, quervernetzt), Crospovidone, Natriumstärkeglykolat, Hydroxypropylcellulose (niedrigsubstituiert), Maisstärke, Polyvinylpyrrolidon, Copolymerisaten von Vinylpyrrolidon mit anderen Vinylderivaten (Copovidone), Hydroxypropylcellulose, Hydroxypropylmethylcellulose, Microkristalliner Cellulose oder Stärke, Magnesiumstearat, Natriumstearylfumarat, Talk, Hydroxypropylmethylcellulose, Carboxymethylcellulose, Celluloseacetatphthalat, Polyvinylacetat, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen eingearbeitet.

Tabletten erhält man z.B. durch Mischen des oder der Wirkstoffe mit ein oder mehreren Hilfsstoffen und anschliessende Verpressung. Die Tabletten können auch aus mehreren Schichten bestehen. Beispiele für Hilfsstoffe sind
- inerte Verdünnungsmitteln wie Mannitol, Sorbitol, Xylit, Saccharose, Calciumcarbonat, Calciumphosphat und Lactose;
- Sprengmitteln wie Croscarmellose Natriumsalz (Cellulose carboxymethylether Natriumsalz, quervernetzt), Crospovidone, Natriumstärkeglykolat, Hydroxypropylcellulose (niedrigsubstituiert) und Maisstärke;
- Bindemitteln wie Polyvinylpyrrolidon, Copolymerisaten von Vinylpyrrolidon mit anderen Vinylderivaten (Copovidone), Hydroxypropylcellulose, Hydroxypropylmethylcellulose, Microkristalliner Cellulose oder Stärke;
- Schmiermitteln wie Magnesiumstearat, Natriumstearylfumarat und Talk;
- Mitteln zur Erzielung des Depoteffektes wie Hydroxypropylmethylcellulose, Carboxymethylcellulose, Celluloseacetatphthalat und Polyvinylacetat; und
- Pharmazeutisch zulässige Farbstoffe wie farbige Eisenoxide

Bei sämtlichen Aspekten der Erfindung handelt es sich beim ANG II-Antagonisten Telmisartan um {4'-[2-n-Propyl-4-methyl-6-(1-methylbenzimidazol-2-yl)-benzimidazol-1-ylmethyl]-biphenyl-2-carbonsäure} oder um Polymorphe oder Salze davon, vorzugsweise das Natriumsalz. Telmisartan befindet sich bereits auf dem Markt, z. B. unter der Bezeichnung Micardis^{®}.
Telmisartan ist beispielsweise in EP-0 502 314 und US 5 591 762 beschrieben. Polymorphe von Telmisartan sind beispielsweise in WO 00/43370, US 6 358 986 und US 6 410 742 beschrieben. Salze von Telmisartan sind beispielsweise in der WO 03/037876 beschrieben.

Beispielsweise wird in WO 03/037876 ausgeführt, dass sich das Natriumsalz von Telmisartan der Formel 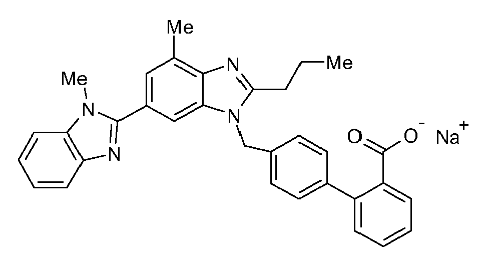 in selektiver Weise in einer kristallinen polymorphen Form durch geeignete Wahl der Herstellungsbedingungen erhalten lässt.
Diese kristalline Form des Natriumsalzes von Telmisartan ist durch den Schmelzpunkt T = 245 ± 5 °C gekennzeichnet (bestimmt durch Differentialscanningkalorimetrie unter Verwendung der Mettler-Toledo DSC82-Vorrichtung; Aufheizgeschwindigkeit: 10° K/min).
Zur Herstellung des Telmisartan-natriumsalzes kann man gemäß einem der beiden folgenden Herstellungsverfahren vorgehen.

Gemäß sämtlichen Aspekten der Erfindung handelt es sich bei dem HMG-CoA-Reduktase-Inhibitor Simvastatin um {2,2-dimethyl-butanoic acid, 1,2,3,7,8,8a-haxahydro-3,7-dimethyl-8-[2-tetrahydro-4-hydroxy-6-oxo-2H-pyran-2-yl)-ethyl-1-naphthalenyl ester}, das beispielsweise unter den Handelsbezeichnungen Zocor^{R} vertrieben wird.
Simvastatin ist beispielweise in EP 0 033 538 und US 4 444 784 beschrieben.

Unter gemeinsamer Verabreichung der beiden Wirkstoffe ist eine zeitlich aufeinanderfolgende oder gleichzeitige Verabreichung zu verstehen, wobei die gleichzeitige Verabreichung bevorzugt wird. Zur aufeinanderfolgenden Verabreichung kann Telmisartan vor oder nach Verabreichung von Simvastatin gegeben werden.
Die Wirkstoffe können oral, bukkal, parenteral, durch Inhalation, rektal oder topisch verabreicht werden, wobei die orale Verabreichung bevorzugt wird. Eine parenterale Verabreichung kann subkutane, intravenöse, intramuskuläre und intrasternale Injektionen sowie Infusionstechniken umfassen.
Die Wirkstoffe können oral in einer Vielzahl unterschiedlicher Dosierungsformen verabreicht werden, d. h. sie können mit verschiedenen pharmazeutisch verträglichen inerten Trägerstoffen zu Tabletten, Kapseln, Pastillen, Bonbons, Pulvern, Sprühmitteln, wässrigen Suspensionen, Elixieren, Sirups und dergl. zubereitet werden. Zu derartigen Trägerstoffen gehören feste Verdünnungsmittel oder Füllstoffe, sterile wässrige Medien und verschiedene nicht-toxische organische Lösungsmittel. Außerdem können derartige orale pharmazeutische Zubereitungen in geeigneter Weise mit Süßstoffen und/oder Geschmackstoffen versehen werden, wobei man sich verschiedener Mittel, die für derartige Zwecke gebräuchlich sind, bedient. Im allgemeinen liegen die erfindungsgemäßen Verbindungen in derartigen oralen Dosierungsformen in Konzentrationsbereichen von etwa 0,5 bis etwa 90 Gew.-%, bezogen auf die gesamte Zusammensetzung, in solchen Mengen vor, dass sie zur Bildung der gewünschten Dosiseinheiten führen. Zu weiteren geeigneten Dosierungsformen für die erfindungsgemäßen Verbindungen gehören Zubereitungen und Vorrichtungen mit kontrollierter Freisetzung, die dem Fachmann geläufig sind. Für die orale Verabreichung können Tabletten mit einem Gehalt an verschiedenen Trägern, wie Natriumcitrat, Calciumcarbonat und Calciumphosphat zusammen mit verschiedenen Sprengmitteln, wie Stärke und vorzugsweise Kartoffel- oder Tapiokastärke, Alginsäure und bestimmten komplexen Silicaten, zusammen mit Bindemitteln, wie Polyvinylpyrrolidon, Saccharose, Gelatine und Gummi arabicum, verwendet werden. Ferner können Gleitmittel, wie Magnesiumstearat, Natriumlaurylsulfat und Talcum oder Zusammensetzungen eines ähnlichen Typs ebenfalls als Füllstoffe in gefüllten Weich- und Hartgelatinekapseln verwendet werden. Hierzu gehören auch Lactose oder Milchzucker sowie hochmolekulare Polyethylenglykole. Sofern wässrige Suspensionen und/oder Elixiere für die orale Verabreichung erwünscht sind, können die Wirkstoffe mit verschiedenen Süßungs- oder Geschmackstoffen, farbgebenden Mitteln oder Farbstoffen und gegebenenfalls Emulgiermitteln und/oder Wasser, Ethanol, Propylenglykol, Glycerin und verschiedenartigen Kombinationen davon vereinigt werden.
Für eine parenterale Verabreichung können Lösungen der Verbindungen in Sesam- oder Erdnussöl oder in wässrigem Propylenglykol sowie sterile wässrige Lösungen der entsprechenden pharmazeutisch verträglichen Salze verwendet werden. Derartige wässrige Lösungen sind gegebenenfalls in geeigneter Weise zu puffern und das flüssige Verdünnungsmittel ist gegebenenfalls mit ausreichenden Mengen an Kochsalz oder Glucose isotonisch zu machen. Diese speziellen wässrigen Lösungen eignen sich insbesondere für die intravenöse, intramuskuläre und subkutane Injektion. Dabei lassen sich sterile wässrige Medien leicht gemäß dem Fachmann bekannten üblichen Techniken erhalten. Beispielsweise wird üblicherweise destilliertes Wasser als flüssiges Verdünnungsmittel verwendet. Das fertige Präparat wird durch ein geeignetes Bakterienfilter, z. B. ein Filter aus gesintertem Glas, Diatomeenerde oder unglasiertem Porzellan, gegeben. Zu bevorzugten Filtern dieses Typs gehören Berkefeld-, Chamberland- und Asbestscheiben-Metall-Seitz-Filter, wobei die Flüssigkeit mit Hilfe einer Saugpumpe in einen sterilen Behälter gesaugt wird. Die erforderlichen Stufen sind während der gesamten Herstellung dieser injizierbaren Lösungen so durchzuführen, dass die Endprodukte in einem sterilen Zustand vorliegen.
Für die transdermale Verabreichung gehören zu den Dosierungsformen der speziellen Verbindungen bzw. Kombinationen beispielsweise Lösungen, Lotionen, Salben, Cremes, Gele, Suppositorien, Präparate mit verzögerter Freisetzung und Vorrichtungen hierfür. Derartige Dosierungsformen umfassen insbesondere die Verbindung(en) und können Ethanol, Wasser, Penetrationsverstärker und inerte Trägerstoffe, z. B. gelbildende Materialien, Mineralöl, Emulgiermittel, Benzylalkohol und dergl., enthalten.

Die hergestellten Darreichungsformen enthalten z.B. ein Äquivalent von 2,5-40 mg, vorzugsweise von 5, 10, 15, 20, 25, 30, 35 oder 40 mg Simvastatin. Simvastatin lässt sich in Tagesdosen von etwa 0,625 mg (oder 0,009 mg/kg Körpergewicht, bezogen auf eine Person mit einem Gewicht von 70 kg) bis etwa 450 mg (6,43 mg/kg Körpergewicht, bezogen auf eine Person mit einem Gewicht von 70 kg) auf oralem Wege, von etwa 20 mg (0,286 mg/kg Körpergewicht, bezogen auf eine Person mit einem Gewicht von 70 kg) auf parenteralem Wege und vorzugsweise in einer Dosis von etwa 1,25 mg (0,018 mg/kg Körpergewicht, bezogen auf eine Person mit einem Gewicht von 70 kg) bis etwa 80 mg (1,428 mg/kg Körpergewicht, bezogen auf eine Person mit einem Gewicht von 70 kg) auf oralem Wege verabreichen. Besonders bevorzugt wird eine orale tägliche Dosierung von etwa 2,5 mg (0,036 mg/kg Körpergewicht, bezogen auf eine Person mit einem Gewicht von 70 kg), etwa 5 mg (0,071 mg/kg Körpergewicht, bezogen auf eine Person mit einem Gewicht von 70 kg), etwa 10 mg (0,143 mg/kg Körpergewicht, bezogen auf eine Person mit einem Gewicht von 70 kg), etwa 20 mg (0,286 mg/kg Körpergewicht, bezogen auf eine Person mit einem Gewicht von 70 kg) oder etwa 40 mg (0,571 mg/kg Körpergewicht, bezogen auf eine Person mit einem Gewicht von 70 kg) oder speziell zu Beginn eine orale tägliche Dosis von etwa 10 mg auf oralem Wege.

Die hergestellten Darreichungsformen enthalten z.B. ein Äquivalent von 20-200 mg, vorzugsweise von 20, 40, 80, 120, 160 oder 200 mg der freien Säure von Telmisartan. Ist der Wirkstoff mit HCTZ oder Chlorthalidone kombiniert, so enthält die Darreichungsform z.B.10-50 mg, vorzugsweise 50, 25 oder 12,5 mg des Diuretikums. Telmisartan oder Polymorphe oder Salze davon lassen sich in einer täglichen Dosis von 10 mg (oder 0,143 mg/kg Körpergewicht, bezogen auf eine Person mit einem Gewicht von 70 kg) bis 500 mg (7,143 mg/kg Körpergewicht, bezogen auf eine Person mit einem Gewicht von 70 kg) auf oralem Wege und von etwa 20 mg (0,286 mg/kg Körpergewicht, bezogen auf eine Person mit einem Gewicht von 70 kg) auf parenteralem Wege, vorzugsweise von 20 mg (0,286 mg/kg Körpergewicht, bezogen auf eine Person mit einem Gewicht von 70 kg) bis 100 mg (1,429 mg/kg Körpergewicht, bezogen auf eine Person mit einem Gewicht von 70 kg) auf oralem Wege verabreichen. Besonders bevorzugt wird eine orale tägliche Dosis von 40 mg (0,571 mg/kg Körpergewicht, bezogen auf eine Person mit einem Gewicht von 70 kg) bis 80 mg (1,143 mg/kg Körpergewicht, bezogen auf eine Person mit einem Gewicht von 70 kg) oder speziell eine Dosis von etwa 80 mg (1,143 mg/kg Körpergewicht, bezogen auf eine Person mit einem Gewicht von 70 kg).
Vorzugsweise beträgt in der pharmazeutischen Kombination das Verhältnis von Simvastatin zu Telmisartan oder den Polymorphen oder Salzen davon 1:100 bis 100:1 (bezogen auf das Gewicht).
In besonders bevorzugten Ausführungsformen wird Simvastatin zusammen mit Telmisartan oder einem Polymorphen oder Salz davon auf oralem Wege in folgenden täglichen Dosen verabreicht: 5 mg Simvastatin und 40 mg Telmisartan (oder ein Polymorphes oder Salz davon); 5 mg Simvastatin und 80 mg Telmisartan (oder ein Polymorphes oder Salz davon); 10 mg Simvastatin und 40 mg Telmisartan (oder ein Polymorphes oder Salz davon); 10 mg Simvastatin und 80 mg Telmisartan (oder ein Polymorphes oder Salz davon); 20 mg Simvastatin und 40 mg Telmisartan (oder ein Polymorphes oder Salz davon); 20 mg Simvastatin und 80 mg Telmisartan (oder ein Polymorphes oder Salz davon). Gemäß einer bevorzugten Ausführungsform enthalten die erfindungsgemäßen pharmazeutischen Zusammensetzungen Simvastatin in einer Menge von 0,625 mg bis 450 mg und Telmisartan in einer Menge von 10 mg bis 500 mg in einzelnen Dosiseinheiten, gegebenenfalls zusammen mit einem oder mehreren pharmazeutisch verträglichen Verdünnungsmitteln und/oder Trägerstoffen.
Gemäß einer weiteren bevorzugten Ausführungsform enthalten die erfindungsgemäßen pharmazeutischen Zusammensetzungen Simvastatin in einer Menge von 1,25 mg bis 80 mg und Telmisartan in einer Menge von 20 bis 100 mg in einzelnen Dosiseinheiten, gegebenenfalls zusammen mit einem oder mehreren pharmazeutisch verträglichen Verdünnungsmitteln und/oder Trägerstoffen.
Eine weitere bevorzugte Untergruppe von erfindungsgemäßen pharmazeutischen Zusammensetzungen enthält Simvastatin in einer Menge von 2,5 bis 20 mg und Telmisartan in einer Menge von 40 mg bis 80 mg in einzelnen Dosiseinheiten, gegebenenfalls zusammen mit einem oder mehreren pharmazeutisch verträglichen Verdünnungsmitteln und/oder Trägerstoffen.
Eine weitere bevorzugte Untergruppe von erfindungsgemäßen pharmazeutischen Zusammensetzungen enthält Simvastatin in einer Menge von 5, 10 oder 20 mg und Telmisartan in einer Menge von 40 oder 80 mg in einzelnen Dosiseinheiten, gegebenenfalls zusammen mit einem oder mehreren pharmazeutisch verträglichen Verdünnungsmitteln und/oder Trägerstoffen.
Wie bereits erwähnt, betrifft die vorliegende Erfindung auch die Verwendung von Telmisartan zur Herstellung einer pharmazeutischen Zusammensetzung zur Behandlung des humanen oder nicht-humanen Säugetierkörpers zur Prophylaxe oder Therapie der vorerwähnten Indikationen bei Verwendung in Kombination mit Simvastatin. Unter dieser Verwendung ist die Herstellung sämtlicher vorerwähnter erfindungsgemäßer pharmazeutischer Zusammensetzungen zu verstehen.

### Beispiele:

### Beispiel 1: Telmisartan, Losartan und Irbesartan binden in vitro nicht an die PPARgamma Ligandenbindungsdomäne

Protein enthaltend die humane PPARgamma-Ligandenbindungsdomäne (LBD) wird als GST-Fusionsprotein in E.coli hergestellt und affinitätschromatographisch gereinigt.
Hierzu wird ein DNA Abschnitt, der für die Aminosäuren 205-505 des humanen PPARgamma2-Transkriptionsfaktors kodiert (vgl. Genbank-Eintrag U79012) über die zusätzlich eingefügten Restriktionsschnittstellen BamH I und Xho I in den Expressionsvektor pGEX-4T-1 (Amersham) subkloniert und die Sequenz des Abschnitts kontrolliert. Die Expression des Fusionsproteins erfolgt im für pGEX Vektoren empfohlenen E.coli-Stamm BL21(DE3) nach Induktion mit 0,2mM IPTG für 4 Stunden bei 25°C. Die Bakterien werden im Anschluss an die Induktion pelletiert und portionsweise in PBS, pH7.4 weggefroren. Nach Aufschluß in einer French Press, wirde das gelöste GST-PPARgamma-LBD-Fusionsprotein mit Hilfe einer GSTrap-Säule (Pharmacia) gereinigt. Die Elution erfolgte durch Zugabe von 20mM reduziertem Glutathion.
Die GST-PPARgamma-LBD-Proteinfraktionen werden mit Hilfe einer HiTrap Desalting-Säule (Pharmacia) entsalzt und die Proteinkonzentration mit einem Standardassay bestimmt.

Protein enthaltend die humane RXRalpha-Ligandenbindungsdomäne (LBD) wird als His tag-Fusionsprotein in E.coli hergestellt und affinitätschromatographisch gereinigt. Hierzu wird ein DNA-Abschnitt, der für die Aminosäuren 220-461 des humanen RXRalpha-Transkriptionsfaktors kodiert (vgl. Genbank-Eintrag NM_002957, nt 729-1457) über die zusätzlich eingefügten Restriktionsschnittstellen BamH I und Not I in den Expressionsvektor pET28c (Novagen) subkloniert und die Sequenz des Abschnitts kontrolliert. Die Expression des Fusionsproteins erfolgt im für pET-Vektoren empfohlenen E.coli-Stamm BL21(DE3) nach Induktion mit 0,2mM IPTG für 4 Stunden bei 25°C. Die Bakterien werden im Anschluss an die Expression pelletiert und portionsweise in PBS, pH7.4 weggefroren. Nach Aufschluß in einer French Press, wird das gelöste His-RXRalpha-LBD-Fusionsprotein mit Hilfe einer HiTrap Chelating-Säule (Pharmacia) gereinigt. Die Elution erfolgte durch eine 500mM Imidazol-Stufe. Die His-RXRalpha-LBD-Proteinfraktionen werden mit Hilfe einer HiTrap Desalting-Säule (Pharmacia) entsalzt und die Proteinkonzentration mit einem Standardassay bestimmt.
a) AlphaScreen
Alpha Screen-Assays wurden erstmals in Ullmann EF et al, Proc Natl Acad Sci USA (1994) 91:5426-5430 beschrieben. Die im Rahmen dieses Beispiel durchgeführten Messungen erfolgten wie bei Glickman JF et al., J Biomol Screen (2002) 7:3-10 beschrieben. Der Assay-Puffer besteht aus 25mM Hepes pH7.4, 100mM NaCl, 1 mM DTT, 0,1% Tween-20, 0,1% BSA. 3nM GST-PPARgamma-LBD-Fusionsprotein, 15nM biotinyliertes LXXLL-Peptid des Cofaktros CBP (entsprechend dem auf Seite 218 von Mukherjee R et al., J Steroid Biochem Mol Biol (2002) 81:217-225 offenbarten Peptid mit einem zusätzlichen N-terminalen Cystein) und jeweils 10pg/ml anti-GST-Akzeptorbeads bzw. Streptavidin-Donorbeads (Applied Biosystems) werden in einem Gesamtvolumen von 12,5µl in Gegenwart von unterschiedlichen Konzentrationen einer Testsubstanz (in DMSO) für 4 Stunden bei Raumtemperatur inkubiert. Die finale DMSO-Konzentration im Assay beträgt 1 % (v/v). Als Hintergrundkontrolle (NSB) dient eine 1 %ige DMSO-Lösung. Die Messung erfolgt am Packard Fusion-Messgerät.

| Konz. / M | **Telmisartan** | | **Rosiglitazone** | |
|---|---|---|---|---|
| | MW | SD | MW | SD |
| NSB | 619 | 21 | 573 | 17 |
| 1,00E-08 | | | 820 | 18 |
| 3,00E-08 | 642 | 41 | 1720 | 48 |
| 1,00E-07 | 606 | 10 | 8704 | 59 |
| 3,00E-07 | 644 | 56 | 27176 | 1232 |
| 1,00E-06 | 677 | 14 | 43233 | 1083 |
| 3,00E-06 | 720 | 35 | 52691 | 3771 |
| 1,00E-05 | 847 | 82 | 56366 | 4303 |
| 5,00E-05 | 1111 | 135 | | |

Im Gegensatz zu Rosiglitazone, einem literaturbekannten PPARgamma-Agonisten mit Bindung in der LBD, findet durch zunehmende Konzentrationen von Telmisartan, Losartan und Irbesartan (Konzentrationen bis 50µM) keine direkte Aktivierung der PPARgamma-LBD und damit verbunden keine signifikante Rekrutierung des LXXLL-Peptids statt.
b) SPA-Assay
Eine Beschreibung des SPA-Assayformats findet sich in Mukheriee R et al., J Steroid Biochem Mol Biol (2002) 81:217-225. Der Assaypuffer besteht aus 20mM Tris pH7.5, 25mM KCI, 10mM DTT, 0,2% Triton X-100). 30nM GST-PPARgamma-LBD-Fusionsprotein, 30nM His-RXRalpha-LBD, anti-GST-Antikörper (1:600, Amersham Pharmacia), 0,25mg Protein A SPA PVT Antibody-binding beads (Amersham Pharmacia), 30nM ³H-markiertes Rosiglitazone werden in einem Gesamtvolumen von 100µl mit Testsubstanzverdünnungen für 5 Stunden bei Raumtemperatur inkubiert.
Als Hintergrundkontrolle (NSB) wird 10µM unmarkiertes Rosiglitazone anstelle des radioaktiven Rosiglitazones zugesetzt, , als Maximalwert (Bmax) wird anstelle einer Testsubstanz das verwendete Lösungsmittel, z.B. DMSO, zugegeben.
Im Anschluss an die Inkubation werden die Testansätze für 5 Minuten bei 2000 Upm in einer Hettich Universal 30Rf-Zentrifuge abzentrifugiert und am Packard TopCount NXT gemessen.

| Konz / M | **Telmisartan** | | **Irbesartan** | | **Losartan** | |
|---|---|---|---|---|---|---|
| | MW | SD | MW | SD | MW | SD |
| NSB | 217 | 9 | 217 | 9 | 217 | 9 |
| Bmax | 911 | 15 | 911 | 15 | 911 | 15 |
| 1,00E-07 | 837 | 49 | 913 | 54 | 915 | 43 |
| 3,00E-07 | 802 | 28 | 810 | 49 | 835 | 11 |
| 1,00E-06 | 818 | 27 | 815 | 51 | 901 | 10 |
| 3,00E-06 | 818 | 20 | 779 | 26 | 814 | 53 |
| 1,00E-05 | 703 | 30 | 723 | 37 | 787 | 46 |
| 3,00E-05 | 691 | 222 | 648 | 40 | 784 | 96 |
| 1,00E-04 | 545 | 18 | 510 | 81 | 611 | 17 |

Im Gegensatz zu direkten PPARgamma-Agonisten, die an die PPARgamma-LBD binden, findet auch bei sehr hohen Überschüssen von Telmisartan, Losartan oder Irbesartan keine konzentrationsabhängige Verdrängung des radioaktiven Rosiglitazones aus der Bindungstasche statt.
c) NMR-Untersuchungen
Im Gegensatz zu einem direkten PPARgamma-Liganden, z.B. Rosiglitazone, findet bei der Messung des ¹⁵N TROSY-Spektrums der PPARgamma-LBD in Gegenwart der Testsubstanzen Telmisartan keine Wechselwirkung der Testsubstanz mit Aminosäuren der Bindungstasche statt. Die Aminosäuren der Bindungstasche weisen in Gegenwart der Testsubstanzen die gleiche Positionierung auf, wie in Abwesenheit eines Liganden.

### Beispiel 2: Herstellung einer stabil transformierten PPARgamma-Reporterzelllinie

Ein DNA Abschnitt, der für die Aminosäuren 205-505 des humanen PPARgamma2-Transkriptionsfaktors kodiert (entsprechend den Nukleotiden 703-1605 der Genbank-Sequenz U79012) wird über zusätzlich eingefügte Restriktionsschnittstellen BamH I und Hind III in die Multiple Cloning Site des Vektors pFA-CMV (Stratagene) eingebaut und die Sequenz verifiziert. Das resultierende Plasmid pFA-CMV/hPPARgamma2-LBD kodiert N-terminal der PPARgamma-LBD im gleichen Leseraster für eine Gal4 DNA-Bindungsdomäne. Zusätzlich kodiert das Plasmid für eine Neomycin-Resistenz.

Die Zelllinie CHO-K1 (ATCC CCL-61) wird mit den Plasmiden pFA-CMV/hPPARgamma2-LBD und pFR-Luc (Stratagene) cotransfiziert. pFR-Luc kodiert für das Luciferase-Gen unter der Kontrolle einer fünffach wiederholten Hefe Gal4-Bindungsstelle. Die Transfektion wird mit Lipofectamin2000 gemäß Herstellerangaben durchgeführt.
Nach der Transfektion werden die Zellen in Medium (Ham's F12 mit 10% fötalem Kälberserum) in Gegenwart von 0,5 mg/ml G-418 kultiviert. Nach sechstägiger Kultivierung werden die Zellen passagiert und für weitere 10 Tage in Kultur gehalten. Die resultierenden Neomycin-resistenten Kolonien werden unter dem Mikroskop gepickt und in 96-Well-Platten vereinzelt und kultiviert. Man erhält verschiedene transformierte Zelllinien mit den enthaltenen Plasmiden (z.B. Klon Nr. 10, 11, 13 etc), die weiter im Kulturmedium gehalten werden.

Die Zelllinien werden hinsichtlich der Induzierbarkeit des Luziferase Gens durch einen PPARgamma-Agonisten, z.B. Rosiglitazone, untersucht und reagieren mit einem gesteigerten Luciferasesignal auf Stimulation durch den PPARgamma-Agonisten.

### Beispiel 3: Telmisartan, Losartan und Irbesartan aktivieren PPARgamma zellulär

Die vom transformierten Klon 11 des Beispiels 2 abgeleitete CHO-K1 Zelllinie wird in 96-well-Flachbodenplatten in einer Dichte von 3x10⁴ Zellen/200µl/well ausgesät und über Nacht in Ham's F-12-Medium mit 10% fötalem Kälberserum und 0,5mg/ml G-418 kultiviert. Nach 24 Stunden wird auf Medium ohne Zusatz von G-418 gewechselt.
Die Testsubstanzen werden mit einem geeigneten Lösungsmittel, z.B. DMSO, auf das 100fache der gewünschten Konzentration gebracht und durch das vorgelegte Medium der Zellkulturplatte 1:100 verdünnt. Als Hintergrundkontrolle dient das verwendete Lösungsmittel, z.B. DMSO, in gleicher Konzentration.
24 Stunden nach Substanzgabe werden die Überstände verworfen und die Zellen je zweimal mit 150µl Waschpuffer (25mM Tricine, 16,3mM MgSO₄, pH7.8) gewaschen. Nach den Waschschritten werden je Testansatz 50µl Waschpuffer mit 150µl Luziferase-Assaypuffer (25mM Tricine, 0,5mM EDTA, 0,54mM NaTPP, 16,3mM MgSO₄ 1,2mM ATP, 0,05mM Luciferin, 56,8mM 2-Mercaptoethanol, 0,1% Trition X-100, pH7.8) zugesetzt. Die Messung der Lumineszenz erfolgt nach fünfminütiger Wartezeit am Packard TopCount NXT. Die Luziferase-Aktivität wird durch die Integration der relativen Luziferaseeinheiten (RLU) der ersten zehn Sekunden nach Start der Messung erhalten.

| Konz / M | **Telmisartan** | | **Irbesartan** | | **Losartan** | | **Rosiglitazone** | |
|---|---|---|---|---|---|---|---|---|
| | MW | SD | MW | SD | MW | SD | MW | SD |
| NSB | 466 | 188 | 466 | 188 | 466 | 188 | 741 | 141 |
| 1,00E-08 | | | | | | | 2761 | 178 |
| 3,00E-08 | | | | | | | 8256 | 708 |
| 1,00E-07 | | | | | | | 35265 | 2947 |
| 3,00E-07 | 760 | 255 | 491 | 70 | 874 | 475 | 86859 | 6139 |
| 1,00E-06 | 2859 | 455 | 657 | 65 | 589 | 70 | 106252 | 30018 |
| 3,00E-06 | 24498 | 2290 | 1028 | 342 | 672 | 88 | 143232 | 14064 |
| 1,00E-05 | 61397 | 7853 | 3292 | 556 | 709 | 163 | 150989 | 24245 |
| 3,00E-05 | 58790 | 2055 | 22133 | 4202 | 3271 | 585 | | |
| 1,00E-04 | | | 29600 | 6936 | 11322 | 1668 | | |

Durch den Angiotensin II Rezeptor Antagonisten Telmisartan erfolgt eine besonders starke Aktivierung des PPARgamma-Pathways in der PPARgamma-Reporterzelllinie. Eine Aktivierung durch andere Angiotensin II Rezeptor Antagonisten wie Losartan und Irbesartan findet erst bei höheren Testkonzentrationen und in einem geringeren Ausmaß statt.

### Beispiel 4: Formulierungsbeispiele

**Tablette 1**

| Durch direkte Verpressung des Telmisartan Natriumsalzes mit Hilfsstoffen und Magnesiumstearat werden Tabletten folgender Zusammensetzung erhalten: | |
|---|---|
| Bestandteile: | mg |
| Telmisartan-Natriumsalz | 41,708 |
| Mannitol | 149,542 |
| Mikrokristalline Cellulose | 50,000 |
| Croscarmellose Natriumsalz | 5,000 |
| Magnesiumstearat | 3,750 |
| Gesamt | 250,000 |

**Tablette 2**

| Durch direkte Verpressung des Telmisartan Natriumsalzes mit Hilfsstoffen und Magnesiumstearat werden Tabletten folgender Zusammensetzung erhalten: | |
|---|---|
| Bestandteile: | mg |
| Telmisartan-Natriumsalz | 83,417 |
| Sorbitol | 384,083 |
| Polyvidon K25 | 25,000 |
| Magnesiumstearat | 7,500 |
| Gesamt | 500,000 |

**Tablette 3**

| Hydrochlorothiazid, Telmisartan Natriumsalz, Sorbitol und rotes Eisenoxid werden in einem Freifallmischer ("Free Fall Blender") gemischt, durch ein 0,8 mm Sieb gesiebt und, nach Zugabe von Magnesium Stearat, in einem Freifallmischer zu einer pulverförmigen Mischung verarbeitet. Diese Zusammensetzung von Wirkstoffen und Hilfsstoffen wird dann mit einer geeigneten Tablettenpresse (z.B. Korsch EKO oder Fette P1200) zu Tabletten verpresst. Es werden Tabletten mit folgender Zusammensetzung hergestellt, wobei die pro Tablette enthaltene Menge Telmisartan Natriumsalz einer Menge von 80 mg der freien Säure des Telmisartans entspricht. | | |
|---|---|---|
| **Inhaltstoff** | **mg/Tablette** | **%** |
| Telmisartan Natriumsalz | 83,417 | 13,903 |
| Hydrochlorothiazid | 12,500 | 2,083 |
| Sorbitol | 494,483 | 82,414 |
| Rotes Eisenoxid | 0,600 | 0,100 |
| Magnesiumstearat | 9,000 | 1,500 |
| **Total** | **600,000** | **100,000** |

Das Telmisartan Natriumsalze der Tabletten der drei Chargen löst sich nach 30 Minuten Rühren (75 rpm) in 900 ml 0,1 M Phosphatpuffer pH 7,5 zu 92 ± 1,5 %, 96 ± 1,8 % bzw. 100 ± 1,0 %. Das Hydrochlorothiazid löste sich nach 30 Minuten in 900 ml 0,1 M HCl (100 rpm) zu 69 ± 6,3 %, 72 ± 2,1 % bzw 78 ± 1,8 %.

### Beispiel 5: Herstellung eines kristallinen Telmisartan-natriumsalzes ausgehend von Telmisartan

Beim Ausgangsmaterial zur Herstellung von kristallinem Telmisartan-natriumsalz kann es sich um die freie Säure von Telmisartan handeln, die nach herkömmlichen Verfahren (z. B. gemäß EP-0 502 314) erhältlich ist.
154,4 g Telmisartan werden in einem geeigneten Reaktionsgefäß in 308,8 ml Toluol vorgelegt, die Suspension wird mit 27,8 g einer 44,68%igen Natriumhydroxidlösung und 84,9 ml Ethanol vereinigt und etwa 30 Minuten auf 78°C erwärmt. Sodann wird das Gemisch filtriert. Gegebenenfalls kann dann, wenn große Mengen an Feststoff im Filter verblieben sind, der Filter mit einem Gemisch aus 61,8 ml Toluol und 15,3 ml Ethanol gewaschen werden.
463,2 ml Toluol werden in einem weiteren Reaktionsgefäß vorgelegt und unter Rückfluss erwärmt. Das gemäß dem vorstehend beschriebenen Verfahren erhaltene Filtrat wird langsam bei der Siedetemperatur zugegeben und gleichzeitig azeotrop destilliert. Nach der vollständigen Zugabe wird die Lösung, die gegebenenfalls durch Waschen des Filters erhalten worden ist, ebenfalls zugegeben und erneut azeotrop abdestilliert. Das Gemisch wird bis zu einer Temperatur von 103°C destilliert. Sodann lässt man die Suspension auf Umgebungstemperatur abkühlen. Die Kristalle werden unter Absaugen filtriert, mit 154,4 ml Toluol gewaschen und bei 60°C in einem Umlufttrockenschrank getrocknet.
Ausbeute: 154,6 g (96 %)
Farblose Kristalle

| | | | | |
|---|---|---|---|---|
| C₃₃H₂₉N₄O₂Na x 0,5H₂O | ber.: | C 72,51 | H 5,72 | N 10,25 |
| | gef.: | C 72,57 | H 5,69 | N 10,21 |

### Beispiel 6: Herstellung von kristallinem Telmisartan-natriumsalz aus Telmisartan-hydrochlorid

### Herstellung von Telmisartan-hydrochlorid:

411 g tert.-Butyl-4'-[[2-n-propyl-4-methyl-6-(1-methylbenzimidazol-2-yl)-benzimidazol-1-yl]-methyl]-biphenyl-2-carboxylat werden in 822 ml Eisessig suspendiert und mit 213 g konzentrierter wässriger Salzsäure (37 %) vereinigt. Das Gemisch wird unter Rückfluss erwärmt. Etwa 640 ml des Lösungsmittels werden abdestilliert. Der verbleibende Rückstand wird langsam mit etwa 620 ml Wasser von 50-60°C vereinigt. Dieses Gemisch wird mit 20 g Aktivkohle (z. B. Norit SX 2 Ultra) versetzt. Das erhaltene Gemisch wird etwa 10 Minuten bei konstanter Temperatur gerührt. Nach der Filtration wird der Rückstand 3-mal mit 25 ml Eisessig und etwa 620 ml Wasser gewaschen. Das erhaltene Filtrat wird erneut auf etwa 50-60°C erwärmt und mit etwa 2 Liter Wasser versetzt. Nach etwa 12-stündigem Rühren bei etwa 23°C werden die gebildeten Kristalle unter Absaugen abfiltriert und 2-mal mit etwa 500 ml Wasser und 1-mal mit etwa 900 ml Aceton gewaschen und sodann bei etwa 60°C getrocknet.
Ausbeute: 367 g (92,5 %)
Farblose Kristalle
Schmelzpunkt: 278 °C

*Herstellung von kristallinem Telmisartan-natriumsalz aus Telmisartan-hydrochlorid:* 55,1 g Telmisartan-hydrochlorid werden in 110,2 ml Toluol, 5,5 ml Wasser und 55,1 ml Isopropanol aufgenommen. Dieses Gemisch wird mit 36,9 g Natriummethoxid (30 % in Methanol) und 2,75 g Aktivkohle (z. B. Norit SX 2 Ultra) vereinigt. Anschließend wird das Gemisch auf etwa 75°C erwärmt. Etwa 50 ml des Lösungsmittelgemisches werden bei konstanter Temperatur innerhalb von etwa 30 Minuten abdestilliert. Die erhaltene Suspension wird filtriert. Der Rückstand wird mit etwa 20 ml Toluol gewaschen. Das Filtrat wird mit etwa 5 ml Wasser und etwa 150 ml Toluol vereinigt. Das erhaltene Gemisch wird unter Rückfluss erwärmt. Dabei werden etwa 150 ml Lösungsmittelgemisch azeotrop abdestilliert (bis zu 102°C). Man lässt das Gemisch 1 Stunde bei 100°C kristallisieren. Die Kristalle werden durch Absaugen abfiltriert, mit etwa 50 ml Toluol gewaschen und bei etwa 60°C getrocknet.
Ausbeute: 53,6 g (99 %)
Farblose Kristalle

| | | | | |
|---|---|---|---|---|
| C₃₃H₂₉N₄O₂Na·0,5H₂O | ber.: | C 72,51 | H 5,72 | N 10,25 |
| | gef.: | C 72,44 | H 5,68 | N 10,20 |

## Patentansprüche

1. Verwendung des Angiotensin II Rezeptor Antagonisten Telmisartan oder eines seiner Salze und des HMG-CoA-Reduktase Inhibitors Simvastatin zur Herstellung eines Arzneimittels für die Behandlung von Personen oder Säugetieren bei denen die Prophylaxe oder Therapie von kardiovaskulären, kardiopulmonalen oder renalen Krankheiten angezeigt ist.

2. Verwendung gemäss Anspruch 1 für die Behandlung von Personen bei denen die Prophylaxe oder Therapie von Hypertonie kombiniert mit Hyperlipidämie oder Atherosklerose angezeigt ist.

3. Verwendung gemäss Anspruch 1 für die Behandlung von Personen bei denen die Therapie von Asthma, Bronchitis oder interstitieller Lungenkrankheiten angezeigt ist.

4. Verwendung gemäss Anspruch 1 für die Behandlung von Personen bei denen Diabetes Mellitus Typ 2 diagnostiziert wurde oder Verdacht auf Prädiabetes besteht, zur Prävention von Diabetes und Prädiabetes, oder zur Behandlung des Metabolischen Syndroms und der Insulin Resistenz in Patienten mit normalem Blutdruck.

5. Verwendung gemäss Anspruch 1 zur Therapie oder Prophylaxe einer hypertensiven Insulinresistenz.

6. Verwendung gemäß Anspruch 1, **dadurch gekennzeichnet dass** bei den zu behandelnden Personen der Nüchternblutzuckerwert 110 mg Glucose pro dl Plasma übersteigt.

7. Verwendung gemäß Anspruch 1, **dadurch gekennzeichnet dass** bei den zu behandelnden Personen der Blutwert für Triglyceride 150 mg/dl überschreitet.

8. Verwendung gemäß Anspruch 7, **dadurch gekennzeichnet dass** bei den zu behandelnden Personen der Blutwert für HDL bei Frauen 40 mg pro dl Plasma, bei Männern 50 mg pro dl Plasma unterschreitet.

9. Verwendung gemäß Anspruch 4, **dadurch gekennzeichnet dass** bei den zu behandelnden Personen der systolische Blutdruck einen Wert von 130 mm Hg und der diastolische Blutdruck einen Wert von 80 mm Hg übersteigt.

10. Verwendung gemäss Anspruch 8, **dadurch gekennzeichnet, dass** Simvastatin in einer täglichen Dosis von etwa 0,009 mg/kg Körpergewicht bis 6,43 mg/kg Körpergewicht auf oralem Wege und Telmisartan oder ein Polymorphes oder Salz davon in einer täglichen Dosis von etwa 0,143 mg/kg bis 7,143 mg/kg Körpergewicht auf oralem Wege verabreicht werden.

11. Verwendung gemäss Anspruch 8, **dadurch gekennzeichnet, dass** Simvastatin in einer täglichen Dosis von etwa 0,286 mg/kg Körpergewicht auf parenteralem Wege und Telmisartan oder ein Polymorphes oder Salz davon in einer täglichen Dosis von etwa 0,286 mg/kg Körpergewicht auf parenteralem Wege verabreicht werden.

12. Verfahren für die Behandlung von Personen bei denen die Prophylaxe oder Therapie von kardiovaskulären, kardiopulmonalen oder renalen Krankheiten angezeigt ist, **dadurch gekennzeichnet dass** ein Arzneimittel verabreicht wird, das den Angiotensin II Rezeptor Antagonisten Telmisartan oder eines seiner Salze und den HMG-CoA-Reduktase Inhibitor Simvastatin enthält.

13. Pharmazeutische Zusammensetzung für die Behandlung von Personen oder Säugetieren bei denen die Prophylaxe oder Therapie von kardiovaskulären, kardiopulmonalen oder renalen Krankheiten angezeigt ist umfassend Telmisartan in Kombination mit Simvastatin gegebenenfalls mit ein oder mehreren Hilfsstoffen.

14. Pharmazeutische Zusammensetzung gemäß Anspruch 13, **dadurch gekennzeichnet dass** die Darreichungsform des Arzneimittels 20-200 mg Telmisartan und 2,5-40 mg Simvastatin enthält.

15. Pharmazeutische Zusammensetzung gemäss Anspruch 14, **dadurch gekennzeichnet dass** das Verhältnis von Simvastatin zu Telmisartan oder einem Polymorphen oder Salz davon 1:2 bis 1:16 (bezogen auf das Gewicht) beträgt.

16. Pharmazeutische Zusammensetzung gemäß Anspruch 13, **dadurch gekennzeichnet dass** die beiden Wirkstoffe zusätzlich mit einem Diuretikum kombiniert sind.

17. Pharmazeutische Zusammensetzung gemäß Anspruch 16, **dadurch gekennzeichnet dass** die Darreichungsform des Arzneimittels 10-50 mg HCTZ oder Chlorthalidone enthält.

18. Pharmazeutische Zusammensetzung gemäss Anspruch 13 zur gleichzeitigen, getrennten oder aufeinanderfolgenden Behandlung mit den Wirkstoffen.
